Europäisches Patentamt

European Patent Office

Office européen des brevets

(19)

(11) Publication number: **0 232 628**
**A1**

(12) # EUROPEAN PATENT APPLICATION

(21) Application number: 86310205.9

(22) Date of filing: 30.12.86

(51) Int. Cl.⁴: **A 01 G 7/00, C 12 M 3/00**

(30) Priority: 04.01.86 GB 8600138
31.03.86 IL 78345
31.03.86 IL 78344

(43) Date of publication of application: 19.08.87
Bulletin 87/34

(84) Designated Contracting States: **AT BE CH DE ES FR GB GR IT LI LU NL SE**

(71) Applicant: **P.B.Ind. Plant Biotec Industries Ltd., Mobile Post, Ashrat 25201 (IL)**

(72) Inventor: **Waters, Roy, 36 Fields Court, Warwick (GB)**
Inventor: **Mutton, Michael Keith, Grafton House§School Road, Alcester (GB)**
Inventor: **Levin, Robert, Kibbutz Beit HaEmek, Mobile Post Maale HaGalil (IL)**
Inventor: **Hirsch, Stanley, Kibbutz Tuval, D.N. Maale HaGalil (IL)**
Inventor: **Denoor, Yoram, Moshav Yodfat, Mobile Post Misgav (IL)**
Inventor: **Poles, Nethanel, Rehov Habanim 33, Ramat Hasharon (IL)**
Inventor: **Weiss, Nesan, Rehov Ben Zvi 38/19, Kiryat Haim (IL)**

(74) Representative: **Anderson, John Robert Browning et al, WALFORD & HARDMAN BROWN Trinity House Hales Street, Coventry, CV1 1NP (GB)**

(54) **Apparatus and method for plant growth and development.**

(57) A plant growth and development system and technique for the deposition of micro-propagated plant material in a cellular growing matrix (46, 202) and to transplanting of plantlets from each cell of such a matrix (46, 202) to respective growing positions in, for example, a nursery seed tray (204). A cellular growing matrix (46, 202) for growing plant tissue culture propagules is also described and claimed.

EP 0 232 628 A1

## APPARATUS AND METHOD FOR PLANT GROWTH AND DEVELOPMENT

### FIELD OF THE INVENTION

The present invention relates to plant growth and development apparatus and more particularly to the deposition of micro-propagated plant material in a cellular growing matrix and to transplanting of plantlets from each cell of such a matrix to respective growing positions in, for example, a nursery seed tray. The present invention also relates to a cellular growing matrix for growing plant tissue culture propagules.

### BACKGROUND OF THE INVENTION

There is described in applicant's Published European Patent Application 0132414, published on March 13, 1985, a method of growing micro-propagated plant material whereby the plant material is supported on a screen over a culture medium. The uniformity and quality of the tissue culture produced by the process described in the aforesaid Published European Patent Application 0132414 is dependent on the evenness of the distribution of propagules over the surface of the medium in the culture vessel.

The conventional procedure for plant tissue culture propagation is widely practiced and extensively reviewed in the literature. For example, current practices are described by P.F. Wetherall, "In Vitro Propagation" (Avery, 1982); B.V. Conger, Ed.; T.A. Thorpe, "Plant Tissue Culture - Methods and

1

Applications in Agriculture" (Academic Press, 1981). Simply viewed, in vitro propagation consists of aseptically removing a portion of a plant and embedding it in a nutrient medium which causes growth and/or proliferation to occur. More particularly, plant tissue culture can be divided into a sequence of three major stages. The objective of the first stage is the initiation of rapidly developing aseptic culture. This culture can be initiated from a number of plant organs.

Typically, explants are surface disinfected and then handled under sterile conditions. After disinfection, explants are rinsed several times with sterile distilled water and cut into desired segments with sterile instruments. The explant is then placed in a culture room in which light quantity, photoperiod, and temperature are controlled.

After approximately six to eight weeks in the culture room the developed explant material is used for the initiation of the multiplication stage of in vitro propagation. Here the developed explant is aseptically removed from its vessel and trimmed and divided if necessary. The tissue is then placed in a culture vessel in a medium with the appropriate hormone composition and balance to induce multiplication and returned to the culture room. After spending approximately another six to eight weeks in the culture room on a multiplication medium, the tissue is aseptically removed from its vessel, divided, transferred to fresh medium, and returned to the culture room. This transfer is either to a growth medium which is formulated to induce development of shoots with or without roots that will

2

withstand the transition of the greenhouse environment or to a multiplication medium once again for further propagation.

The ability to repeatedly recycle tissue culture through the multiplication stage (Stage II) accounts for the rapid increase which is one of the main advantages of in vitro propagation. This procedure, however, is a very labor intensive activity. It has been estimated that as much as 60-65 per cent of the cost of producing plants in vitro can be attributed to wages, salaries and associated costs. (W. C. Anderson, G.W. Meagher and A.G. Nelson, "Cost of Propagation Broccoli Plants through Tissue Culture", Hortscience 12: 543-544, 1972); (A. Donnnan, Jr., S. E. Davidson and C. L. Williams, "Establishment of Tissue Grown Plants in the Greenhouse Environment", Proc. Fla. State. Hort. Soc. 91: 235-237, 1978). Consequently, there have been various attempts to reduce labor related expenses.

One such successful attempt is described in the aforementioned Published European Patent Application No. 0132414, the teachings of which are incorporated herein by reference. It has been found by applicant that the use of an undifferentiated screen for support of plant material over a culture medium is not particularly suitable for mechanized transplanting of plantlets therefrom.

4 . 0232628

## SUMMARY OF THE INVENTION

The present invention seeks to provide a mechanized system and technique for plantlet growth and development and subsequent transplanting.

There is thus provided in accordance with a preferred embodiment of the present invention a plant growth and development system including a first stage cellular growth matrix comprising a liquid permiable bottom support surface and a wall structure defining a first array of individual growing cells above the bottom support surface, the individual growing cells being separated from adjacent cells in the first array by a first distance, a second stage growth support defining a second array of individual plantlet growth locations, the individual growth locations being separated from adjacent growth locations in the second array by a second distance greater than the first distance, apparatus for providing a generally even distribution of plant propagules onto the first stage cellular growth matrix in engagement with a growth medium, apparatus for mechanically removing individual plantlets growing in the first array of growing cells from the individual growing cells and transplanting them to corresponding individual growing locations on the second stage growth support.

There is also provided in accordance with a preferred embodiment of the present invention a plant growth and development process comprising the steps of:

providing a first stage cellular growth matrix comprising a liquid permiable bottom support surface and a wall

4

structure defining a first array of individual growing cells above the bottom support surface, the individual growing cells being separated from adjacent cells in the first array by a first distance;

providing a second stage growth support defining a second array of individual plantlet growth locations, the individual growth locations being separated from adjacent growth locations in the second array by a second distance greater than the first distance;

providing a generally even distribution of plant propagules onto the first stage cellular growth matrix in engagement with a growth medium;

mechanically removing individual plantlets growing in the first array of growing cells from the individual growing cells and transplanting them to corresponding individual growing locations on the second stage growth support.

According to a preferred embodiment of the invention, the cells are each configured as inverted truncated pyramids to facilitate automated mechanical removal of plantlets therefrom.

Further in accordance with a preferred embodiment of the invention, the apparatus for mechanically removing and transplanting comprises:

apparatus for transferring plantlets from cells in a first stage cellular growth matrix, which cells are arranged in a first regular grid-like array, to respective planting locations in a second stage growing support, the planting locations being arranged in a second regular grid-like array of which the pitch of the centers of the locations is greater than the pitch of the centers of the cells of the first array, the apparatus comprising:

a carrier having a plurality of probes arranged with their centers spaced apart in a regular grid-like array having a pitch substantially the same as that of the locations in the second array;

apparatus for effecting relative bodily movement between the carrier and the first and second arrays, whereby the probes can be aligned first with cells of the first array and then with respective positions in the second array; and

apparatus for effecting extraction of the contents of respective cells of the first array, the retention of said contents on the respective probes until the probes are aligned with respective positions in the second array, and finally the discharge of the contents into the respective positions in the second array.

According to one embodiment of the invention, the apparatus for effecting relative bodily movement comprises apparatus for displacing the carrier along first, second and third perpendicular axes.

According to an alternative embodiment of the invention, the apparatus for effecting relative bodily movement comprises apparatus for displacing the carrier along at least first and second perpendicular axes and apparatus for indexing the first and second arrays along a third axis perpendicular to the first and second axes.

There is also provided in accordance with the present invention a multiple stage plant growth and development system including a first stage cellular growing matrix comprising a screen bottom and a grid arrangement of upstanding walls defining an array of compartments over the screen body, apparatus for providing an even distribution of plant propagules over the first stage cellular growing matrix whereby generally a desired number of plantlets are caused to develop in the first stage growing matrix and apparatus for automatically removing plantlets from compartments in the first stage cellular growing matrix and locating them in compartments in a second stage growing tray of larger scale than the first stage growing matrix.

Additionally in accordance with a preferred embodiment of the invention there is provided a technique for multiple stage plant growth and development including the steps of providing a first stage cellular growing matrix comprising a screen bottom and a grid arrangement of upstanding walls defining an array of compartments over the screen body, providing an even distribution of plant propagules over the first stage cellular growing matrix whereby generally a desired number of plantlets are caused to develop in the first stage growing matrix and automatically removing plantlets from compartments in the first stage cellular growing matrix and locating them in compartments in a second stage growing tray of larger scale than the first stage growing matrix.

Further in accordance with a preferred embodiment of the invention, there is provided a method for obtaining an even distribution of plant propagules on a first stage cellular growing matrix disposed within a vessel including an upper chamber and a lower chamber, the method including the steps of introducing a liquid mixture containing plant tissue culture propagules into the upper chamber while the vessel is sealed at the bottom thereof, providing a stream of air bubbles from an air inlet port, the bubbles rising in the liquid through an effective area which covers a substantial portion of the cross-section of the vessel to effect agitation and mixing of the propagules in the liquid in order to form a substantially homogeneous suspension thereof, and allowing the liquid to drain through the matrix so as to deposit the suspended propagules in substantially even distribution thereon.

The invention also provides apparatus for obtaining an even distribution of plant propagules on a first stage growing matrix disposed in a sealable vessel having upper and lower chambers separated by the growing matrix, the vessel comprising a controllable inlet port for introducing a liquid mixture containing plant tissue culture propagules, a controllable drain port and a controllable air inlet port which leads to an air-bubble distributing sparger.

The present invention provides a sterilizable vessel which can either be opened or closed and sealed, containing a removable, sterilizable, substantially flat fine growing matrix completely separating the vessel into an upper portion containing an inlet port and valve for a plant propagule/liquid mixture and preferably also containing an air pressure relief valve, and a lower portion containing a sintered steel sparger base under the screen, a drain port and an air inlet valve under the sparger.

8

In one preferred embodiment of the present invention the vessel is roughly square in shape with internal dimensions of about 8 cm on a side, and formed from two separable elements, a top element which accounts for about two-thirds of the volume of the vessel and which can be raised and lowered by a pneumatic piston and a bottom element which is fixed in place. The matrix is fixed to a rigid frame, the edges of which rest on and extend over the rim of the lower chamber element such that when the vessel is closed and held under pressure by the pneumatic piston, the vessel is sealed water tight with the screening element in place between the upper and lower chamber of the vessel.

The matrix and frame are all preferably made of relatively inert sterilizable material such as stainless steel for the vessel and nylon for the screen and frame.

As already indicated, the invention is primarily useful in the production of tissue culture derived plantlets by automated procedures. After the plant tissue is aseptically cut and/or divided in bulk, it must be mechanically transported to a culture vessel and aseptically plated on a medium in that vessel, in such a manner as to facilitate growth and development of the tissue. This is accomplished by suspending a fixed amount of plant tissue in a liquid and volumetrically dispensing this suspension to the apparatus described hereinbelow, which distributes the plant tissue evenly over the matrix, over a culture vessel filled with either solid or liquid medium so that the plant material finally rests on top and in good contact with a medium base as described in Published European Patent Application No. 0132414.

Another advantage of the present invention is that it can eliminate the need for a holding tank in an automated micropropagation procedure such as that described in Published European Patent Application No. 0132414. In the process described therein, an even distribution of plant propagules in liquid is obtained by constantly stirring the propagule/liquid suspension in a sterile holding tank. Since in the present invention an even distribution is obtained in the dispensing chamber, this holding tank is no longer needed in an automated micro-propagation procedure such as that described in said Published European Patent Application No. 0132414.

Further in accordance with a preferred embodiment of the present invention there is provided a matrix structure for growing plant tissue culture propagules comprising a regular grid-like array of solid walled cells open at the top and bottom and sized to maintain plantlets developed in each cell in uniform and fixed relationship to one another.

Thus, in its preferred embodiments, the present invention provides for an insert for a plant tissue culture vessel composed of a regular grid-like array or matrix of solid walled cells, open at the top and bottom, typically 7 mm high and 7 mm wide, each cell being preferably shaped like an inverted truncated pyramid, that is, with the opening at the top being substantially larger than the opening at the bottom and with the sides of the cell sloping inwards, the insert being sterilizable and being disposed in the culture vessel at the level of the top of the medium filling the vessel and in good contact therewith.

Preferably the cells are each configured as inverted truncated pyramids and have a width of about 5 to about 12 mm. The matrix structure can be separately manufactured and then positioned on the surface of the screen of the culture vessel or can be produced and distributed already attached at its base to a flat fine sieving and support element having a mesh size which permits the passage of a culture medium therethrough while retaining tissue culture propagules on the surface thereof.

Thus, the cell structure is preferably made of sterilizable material such as nylon and is integrally formed with a plastic sieving and support element.

In a preferred embodiment of the present invention there is provided a sterilizable plant tissue culture vessel characterized by a substantially flat fine sieving and support matrix element elevated above an inner bottom surface of the vessel, the vessel being substantially sealable and adapted for growing plant tissue cultures supported on the surface of said sieving and support element in optimal contact with culture medium filling the vessel to the level of said sieving and support element, the sieving and support element being sized to permit the passage of said culture medium therethrough while retaining said tissue culture on the surface thereof, and being provided on the surface thereof with a cell structure for growing plant tissue culture propagules comprising a regular grid-like array of solid walled cells open at the top and bottom and sized to maintain plantlets developed in each cell in substantially

11

uniform and fixed relationship to each other.

Thus, an embodiment of the present invention relates to a regular grid-like array or matrix of open cells, shaped like inverted truncated pyramids into which sterile plant tissue can be placed so that the material lies on top of, and in good contact with, a medium base, and such that the plant material in each cell can develop into a plantlet or a cluster of plantlets, each of which is physically separated from all others in a fixed position in the culture vessel.

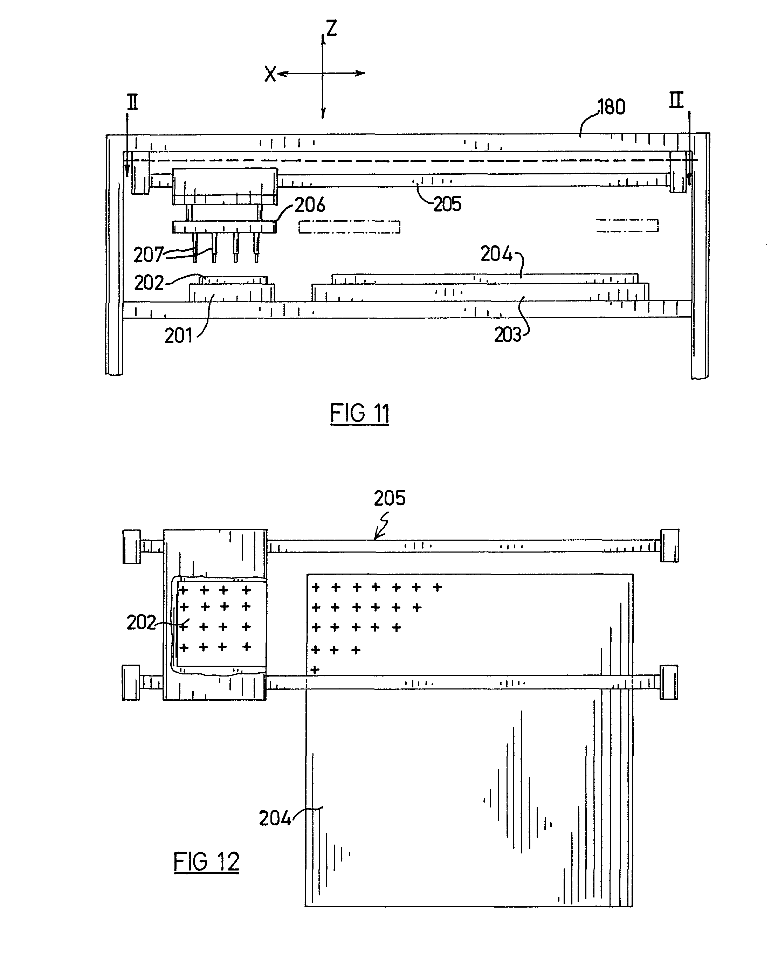

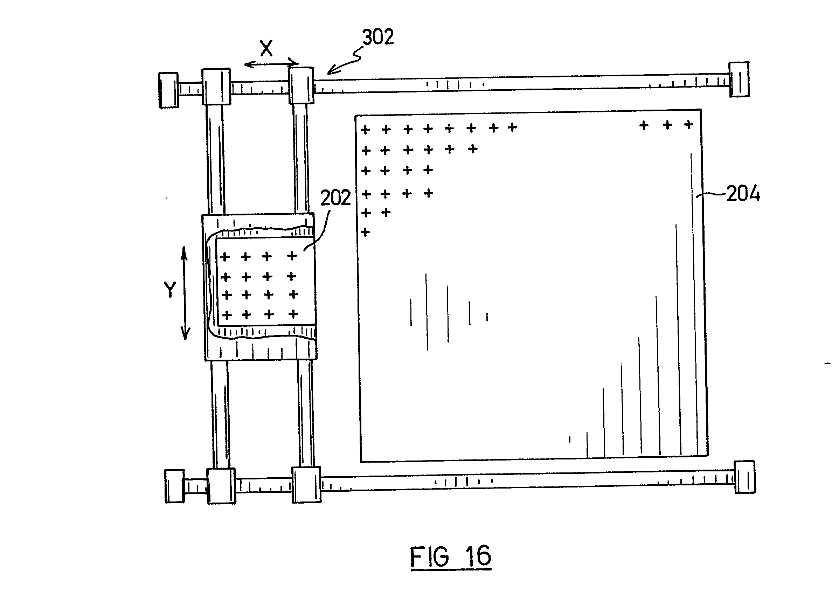

Such a matrix insert for a culture vessel has a number of uses in plant tissue culture propagation, but it is especially applicable to processes related to automated plant micropropagation. For example, it facilitates the operation of a micropropagation transplanting apparatus such as that described herein in connection with Figs. 11-19, since the transplanter can only pick up plantlets which are separated from one another and which occupy fixed positions in the culture vessel. Further, when used in conjunction with a process for at least semi-automated plant tissue culture of the type described in Published European Patent Application 0132414, it allows plantlets produced by this process to be transplanted by the type of machine described herein in connection with Figs. 11-19, which in turn provides an automated micropropagation system encompassing both greenhouse and laboratory.

A further advantage of the matrix is in facilitating the removal of the plantlets from the culture vessel for shipping.

Yet another advantage of the matrix is that when an agar overlay is employed, the tapering walls of the cells do not allow agar plugs holding the plantlets to fall out should the screen be removed to facilitate transplanting by separating the roots entangled in the screen from the plantlet.

There is also provided in accordance with a preferred embodiment of the invention apparatus for transferring the contents of open cells arranged in a first regular grid-like array in the matrix to respective positions arranged in a second regular grid-like array of which the pitch of the centers of the positions is greater than the pitch of the centers of the cells of the first array by a ratio substantially equal to a multiple, comprising a carrier having a plurality of probes arranged with their centers spaced apart in a regular grid-like array having a pitch substantially the same as that of the positions in the second array; means for effecting relative bodily movement between the carrier and the first and second arrays, whereby the probes can be aligned first with cells of the first array and then with respective positions in the second array; means for effecting extraction of the contents of respective cells of the first array, the retention of said contents on the respective probes until the probes are aligned with respective positions in the second array, and finally the discharge of the contents into the respective positions in the second array.

This apparatus enables plants to be extracted simultaneously from a plurality of cells of a first stage growing matrix by a plurality of probes arranged in a fixed pattern and then transplanted in a required grid-like array of positions at a pitch greater in both the length and breadth directions than the pitch of the cells on the growing matrix.

According to another aspect thereof, the invention provides apparatus for transferring the contents of a cell, having an open upper end and a porous or apertured lower end, from a first position, to a second position, by using a tubular probe, the apparatus comprising means for effecting, in succession, relative bodily movement between the probe and means defining the first and second positions, whereby the probe can be aligned with the mouth of a cell in the first position and then with the second position; means to apply a pressure jet of compressed air or other fluid to the cell beneath the contents thereof when the probe is aligned with the mouth of the cell in the first position, thereby to eject the contents of the cell into the probe, and means to apply a pressure jet of compressed air or other fluid to the probe when the latter has been aligned with the second position, thereby to eject the cell contents into said second position.

The tubular probe may have a downwardly-facing frusto-conical chamber, tapering upwardly, in its lower or mouth end, whereby the contents of a cell above which the probe is vertically aligned will be ejected upwardly into the chamber and wedged therein as a result of the aforesaid pressure jet applied beneath the cell. The upper end of the chamber may have at least one vent hole therein through which air displaced by the upward movement of the contents of the cell is vented.

The tubular probe may include a plunger or ejector pin which is retracted to a position above the upper end of the frusto-conical chamber before the contents of a cell are ejected upwardly into the chamber and which is moved downwardly to eject the contents of the cell from the frusto-conical chamber into the transplanting position by the aforesaid pressure jet applied to the probe when the latter has been aligned with the second or transplanting position. The plunger or ejector pin may be biased upwardly into its retracted position by a return spring.

Suction pressure may be applied to the probe simultaneously with the application of the aforesaid pressure jet beneath the cell either to retract the plunger or ejector pin, where a return spring is not provided, or to assist with the upward movement of the contents of the cell into the frusto-conical chamber, and their retention in the chamber, where a plunger or ejector pin is not provided.

## BRIEF DESCRIPTION OF THE DRAWINGS

The present invention will be understood and appreciated more fully from the following detailed description taken in conjunction with the drawings in which:

Fig. 1 is a front view of apparatus constructed and operative in accordance with an embodiment of the invention with the apparatus vessel in the open state;

Fig. 2 is a partial cross-sectional view to a larger scale of the lower vessel portion of the apparatus of Fig. 1;

Fig. 3 is a partial cross-sectional view to a larger scale of the upper vessel portion of the apparatus of Fig. 1;

Fig. 4 is a top view of the apparatus in cross-section along plane IV - IV of Fig. 1;

Fig. 5 is a top view of a first stage growing matrix and its frame;

Fig. 6 is a side view of part of the apparatus, in partial cross-section along plane VI - VI of Fig. 1, showing the vessel in the closed state;

Fig. 7 is a top view of the cell structure of the first stage growing matrix according to the invention;

Fig. 8 is a view in partial cross-section along plane II-II of the cell structure as shown in Fig. 7;

Fig. 9 is an elevational view in partial cross-section of the tissue culture vessel according to the invention and with the first stage growing matrix inserted therein;

Fig. 10 is a top view of the tissue culture vessel with the lid removed and the first stage growing matrix in position;

Fig. 11 is a front elevation illustration of transplanting apparatus in accordance with the present invention;

Fig. 12 is a partially cut away top view illustration in the direction II in Fig. 11;

Fig. 13 is a diagram showing, in plan, part of a transplanting tray shown in Fig. 12 and superimposed thereon, for explanation only, a cellular first stage growing matrix;

Fig. 14 illustrates a vertical section of a bed plate supporting a cellular first stage growing matrix, a probe carrier and a plurality of probes in a preferred embodiment of the apparatus according to the invention;

Figs. 15A and 15B are respective front elevation and end view illustrations of transplanting apparatus in accordance with an alternative embodiment of the present invention;

Fig. 16 is a partially cut away top view illustration taken along the line II-II in Fig. 11A;

Fig. 17 illustrates a vertical section of a bed plate supporting a cellular first stage growing matrix, a probe carrier and a plurality of probes in an alternative preferred embodiment of the apparatus according to the invention;

Fig. 18 illustrates a vertical section of a bed plate supporting a cellular first stage growing matrix, a probe carrier and a plurality of probes in a further alternative embodiment of the apparatus according to the invention; and

Fig. 19 is a block diagram functional illustration of the operation of the apparatus of Figs. 15A - 17.

## DETAILED DESCRIPTION OF PREFERRED EMBODIMENTS

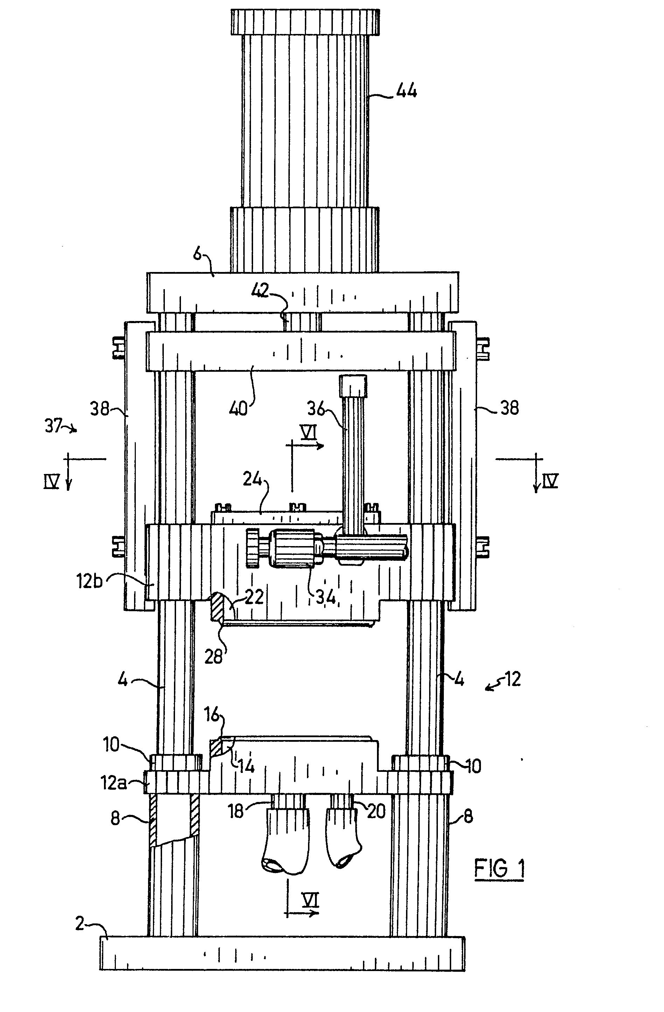

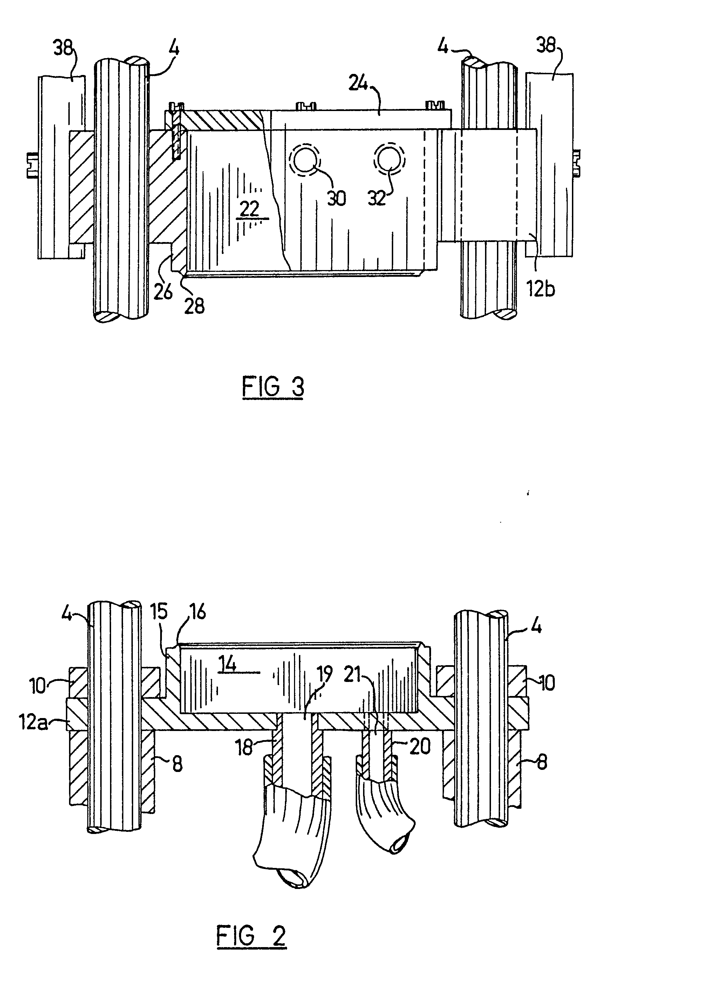

Referring now to the drawings, there is seen in Fig. 1 apparatus for plant growth and development constructed and operative in accordance with an embodiment of the present invention comprising a base 2, onto which are fixedly mounted two columns 4 connected on their upper ends by a cross member 6. Seated on two sleeves 8 slipped over the columns 4, and held in position by clamping rings 10, is the lower stationary portion 12a of a sealable two-part vessel 12 preferably made of stainless steel. This lower portion 12a is shown at a larger scale in Fig. 2 and is seen to comprise a lower chamber 14 defined by a recess of, as seen from above, a substantially square cross section.

The rim 15 of the chamber 14 is provided with a knife edge-type sealing lip 16 surrounding the chamber 14 on all sides. Attached to the bottom of chamber 14 are, in the center, a first rubber-tube connector 18 constituting a drain port 19 and, somewhat off center, a second rubber-tube connector 20 constituting an air inlet port 21, the first serving as a connector for a drain tube leading to a sink or the like, the second as a connector to a source of compressed air. Both tubes are controllable by pinch cocks or valves (not shown).

Fig. 3 shows the upper, movable portion 12b of the vessel 12, comprising an upper chamber 22 constituted by an opening of substantially square cross-section, and tightly covered by a transparent cover plate 24 made of Perspex or a similar material.

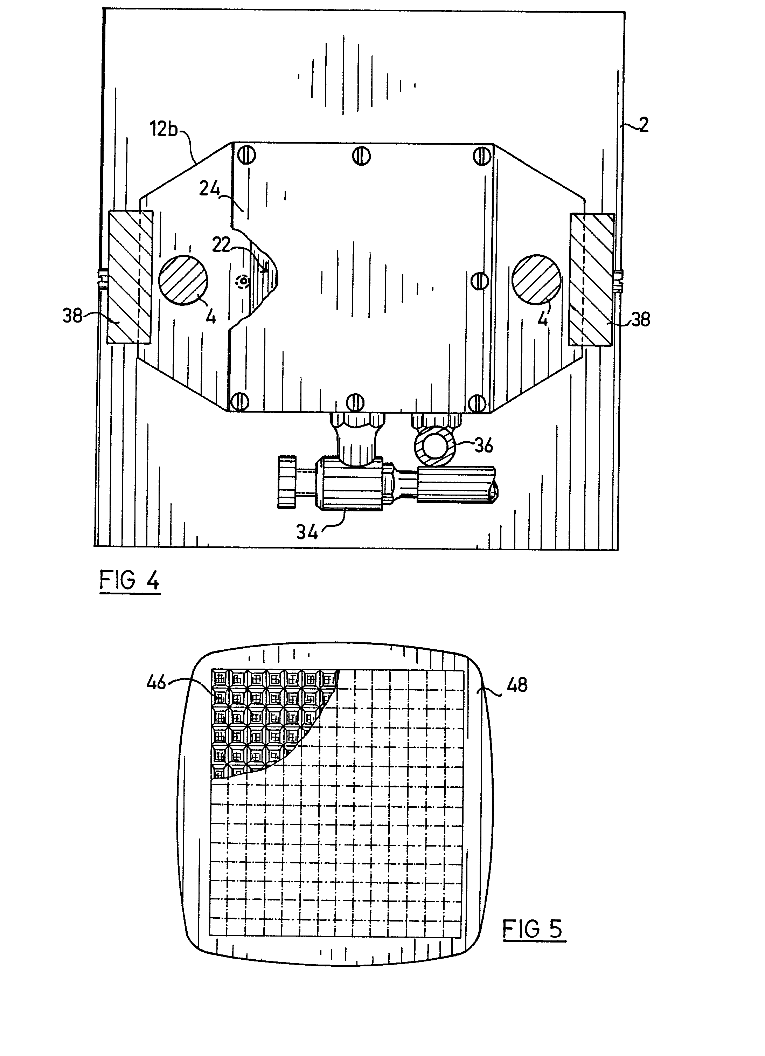

The rim 26 of the chamber 22 is provided with a knife-edge type sealing lip 28 surrounding the rim, similar to the sealing lip 16 of the lower vessel portion 12a. Two threaded holes, 30 and 32, lead into chamber 22, the first, 30, serving to connect a valve 34 controlling the inflow of the above-mentioned plant propagule/liquid mixture, and the second, 32, for connection of an air relief valve 36, the purpose of which will become apparent hereinbelow. Valves 34 and 36 are illustrated in Figs. 1 and 4.

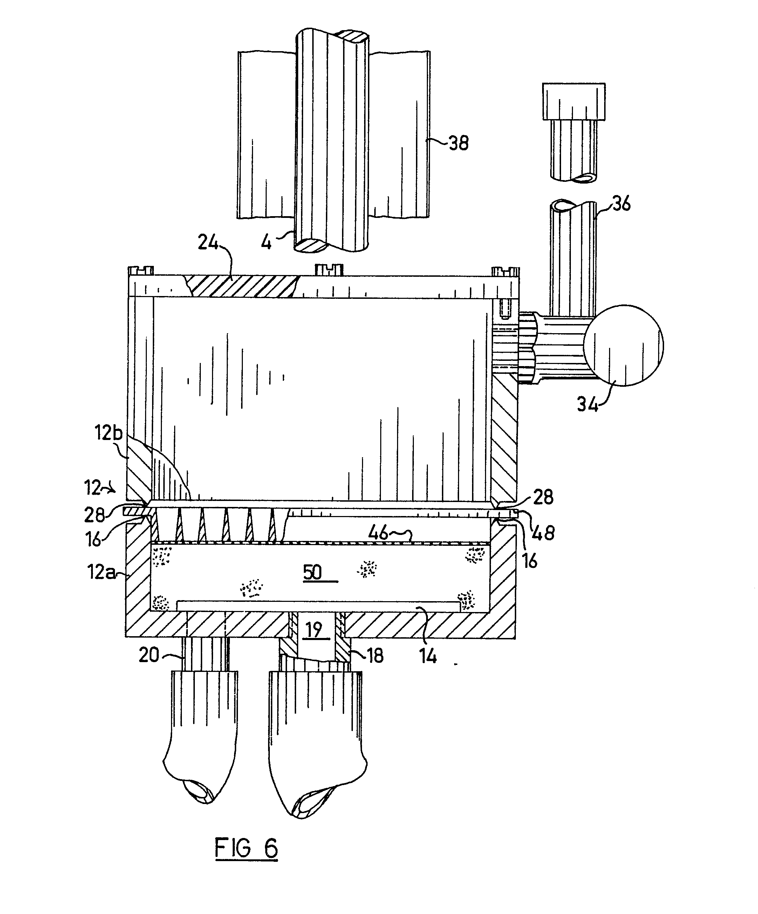

The upper vessel portion 12b is slidably mounted on the columns 4. At the same time it constitutes the lower cross member of a frame 37 (Fig. 1) comprising two uprights 38 to the lower ends of which it is fixedly attached, and an upper cross member 40 (Fig. 1) to which is attached the piston rod 42 of a pneumatic cylinder 44 mounted on the cross member 6. By actuating the cylinder 44, the frame 37 can be raised, as seen in Fig. 1, or lowered, so that the upper vessel portion 12b approaches the lower portion 12a as seen in Fig. 6, to be explained further below.

Fig. 4 shows the outline of the upper vessel portion 12b as seen in top view, the outline of the lower vessel portion 12a being identical.

A first stage growing matrix 46, and its frame flange 48 are shown in Fig. 5. Another view of this matrix is afforded in Fig. 6, which will be explained below.

Fig. 6 illustrates the two-part vessel 12, in the closed, working position in which, by actuating the pneumatic cylinder 44 as mentioned above, the upper vessel portion 12b has

19

been moved downwards and brought to bear against the flange 48 of the matrix 46, which has been inserted into the lower chamber 14. The purpose of sealing lips 16 and 28 is now clear: being relatively sharp, they slightly dent the surfaces of the nylon flange 48, thus producing a tight seal.

Further seen in Fig. 6 is a sparger 50 typically comprising a sintered, porous metal block with a mean pore size of about 35 microns, the purpose of which is to provide an even flow of minute air bubbles through the above-mentioned plant propagule/liquid mixture, thereby ensuring a uniform distribution of the propagules in the liquid, as explained below.

Broadly, the preferred process of an embodiment of the present invention is as follows: The vessel 12, which has been sterilized and maintained in a sterile atmosphere, is closed and sealed by the pneumatic piston, the inlet valve 34 to the upper vessel portion 12b is opened, and a fixed amount of sterile plant material and liquid is pumped into the vessel 12 so that the vessel is nearly filled. The drain port 19 is closed. At the same time, the air valve 36 is opened. Liquid now fills the entire vessel 12 both above and below the matrix, however, the plant material is retained by the matrix 46 in the upper chamber.

Sterile air, under a pressure of 2-3 atmospheres, rises evenly from the sparger 50 in small bubbles, thoroughly mixing the plant material in the liquid above the screen 46. After about 20 seconds of mixing, the air valve 36 is closed and the drain valve is opened. The fluid slowly drains from the vessel 12 leaving the plant material evenly distributed on the matrix.

The vessel 12 is then opened by pneumatically raising the upper portion 12b, and the matrix 46 and its frame 48 are aseptically removed and placed in a culture vessel on top of and in good contact with a medium base which is the optimal configuration for growth and development of the tissue into plantlets. The plantlets are then transplanted to transplanting trays by apparatus and using a technique which is described hereinbelow.

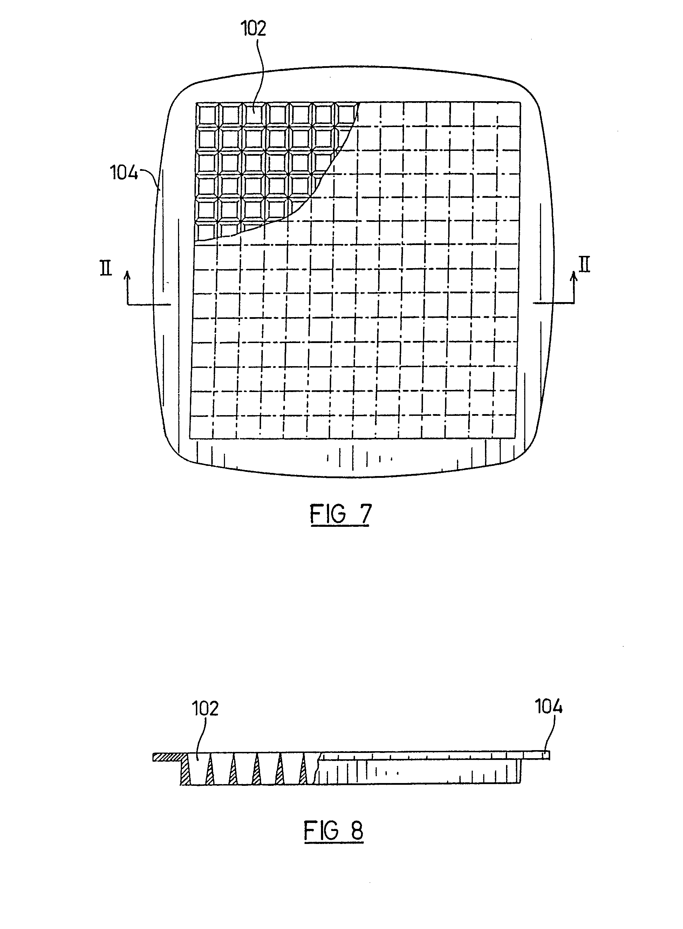

Referring again to the drawings, there is seen in Figs. 7 and 8 the matrix structure according to the invention, advantageously made of a sterilizable material such as one of the nylon groups of plastics, comprising a grid-like array of cells 102 having a substantially square cross-section and being open at the top. The cell walls are solid and converge downwardly such that the cells define inverted truncated pyramids, with each two adjacent walls of each two adjacent cells 102 meeting at the upper surface of the matrix to form a substantially sharp edge, as is clearly seen in Fig. 8. The upper surface of the matrix is provided with a plane rim 104 which fits the tissue culture vessel, discussed further below, with clearance.

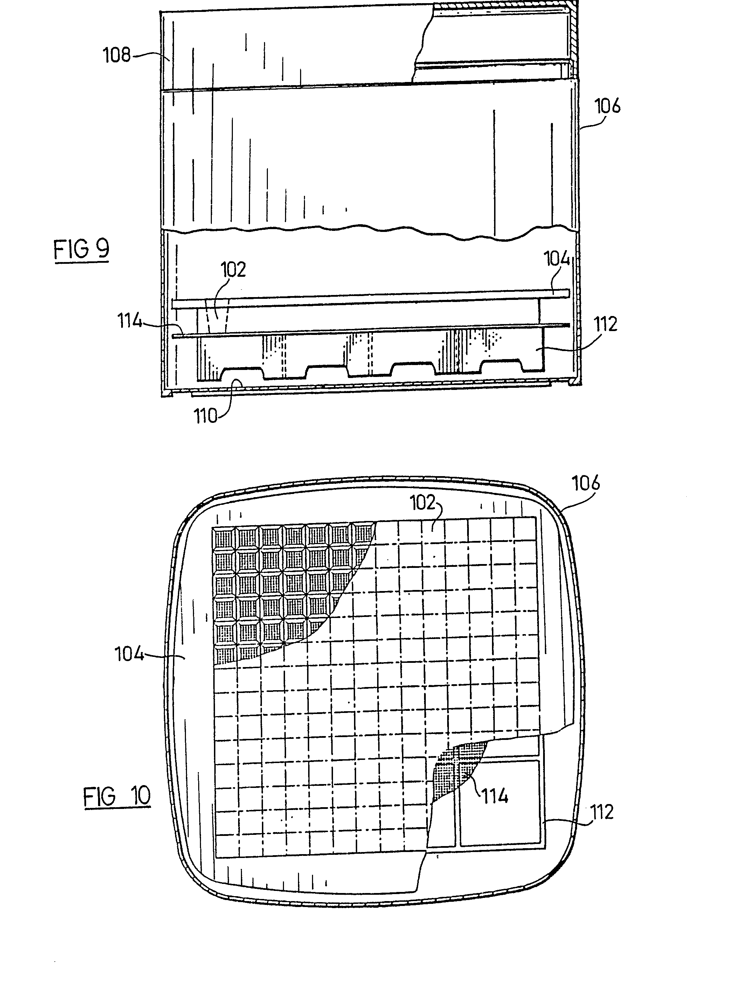

In Figs. 9 and 10, the cell structure according to an embodiment of the invention is shown in position inside the culture vessel 106, the top of which is shown as closed by means of a lid 108. On the bottom 110 of the vessel 106, is disposed a grating-like supporting member or pallet 112, about 1 cm high, on which rests a substantially flat nylon screen 114 which, as has been explained above, allows the culture medium to pass through, but has a mesh fine enough to retain the tissue culture inside the cells 102 of the cell structure mounted on the screen 114. A

21

preferred mesh size is 500 to 700 microns, an acceptable mesh size being 250 - 4000 microns.

While in the embodiment shown the screen 114 is a separate entity, a variant can be envisaged in which the screen 114 is an integral part of the matrix, being, e.g., molded to the bottom surface thereof.

Reference is now made to Figs. 11 - 14 which illustrate transplanting apparatus constructed and operative in accordance with a preferred embodiment of the present invention.

Referring now to Figs. 11 and 12, it is seen that the transplanting apparatus comprises a frame 180. An indexable table 201 is mounted onto frame 180. One or more first stage growing matrices 202 may be mounted onto indexable table 201. Alongside the table 201, there may be provided an endless belt conveyor or bed 203 on which may be supported transplanting trays 204 or an array of compost blocks.

Above the table 201 and the conveyor or bed 203, there is a gantry 205 on which is disposed a carrier 206 having a plurality of probes 207 extending vertically downwardly therefrom and supplied by a pneumatic system, not shown in Figs. 11 and 12. The carrier 206 can be displaced back and forth along an axis designated X between positions above the table 201 and the conveyor or bed 203 and up and down along an axis designated Z towards and away from the table 201 and the conveyor or bed 203.

The cellular matrices 202 are, in this example, each divided into 256 open cells in a 16 x 16 arrangement as viewed in plan. Each transplanting tray 204, or similar arrangement of

0232628

compost blocks, typically has 256 planting positions or divisions arranged in a 16 x 16 arrangement. The side dimension of each cell of the matrix 202 is approximately 5 mm whereas the planting positions in the transplanting tray are arranged at approximately 20 mm spacings.

Thus it may be appreciated that in transplanting growing plants from a matrix 202 to the transplanting tray 204, the plants have to be moved apart in both the X and Y directions through four-fold distances. This is accomplished by extracting the plants from matrix 202, and transferring them to tray 204, by using a system of probes 207, and discharging the plants from the probes 207 at the required positions in the tray 204.

The probes 207 are arranged side-by-side in a grid-like array with the upright axes of the probes 207 spaced apart from each other at substantially the same distances as are the centerlines of the required planting positions in the transplanting tray 204. Thus the probes are arranged with their upright axes spaced apart in a grid-like array at spacings of approximately 20 mm in the X and Y directions.

In Fig. 11 four probes 207 are shown positioned side-by-side in a row. However, in fact, the illustrated embodiment comprises an arrangement of 4 x 4 probes 207.

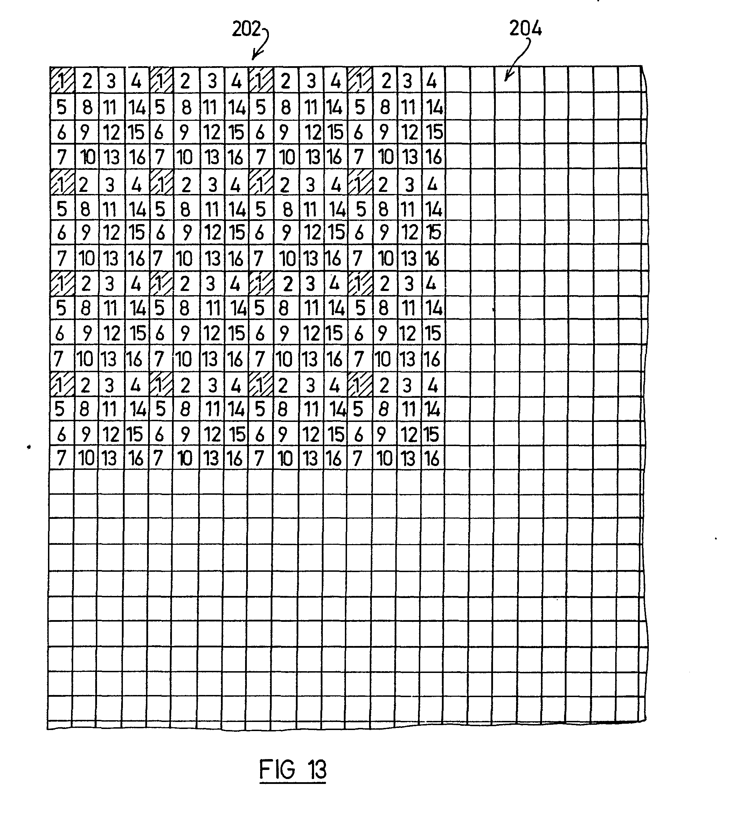

In Fig. 13, one matrix 202 is shown superimposed on part of a transplanting tray 204. This does not occur in reality and has been illustrated only to explain the transplanting method. The 256 cells of the matrix 202 are numbered "1" to "16" in sixteen blocks each of sixteen cells. Sixteen planting positions in the transplanting tray 204 and the corresponding positions of the sixteen probes 207 are indicated by the shaded sixteen cell positions 201.

Relative indexing of the table 201 and X-axis displacement of the array of probes is performed to bring the sixteen cells "1" into vertical alignment beneath each of the sixteen probes 207. The plants in the sixteen cells "1" are extracted, as will be explained later, and are carried by the respective probes 207 into vertical alignment above sixteen planting positions "1" in the transplanting tray 204.

Appropriate X-axis displacement of the probe carrier 206 and Y-axis movement of the conveyor or bed 203 is performed to enable this to be done. The plants are then planted in compost in the transplanting tray or blocks 204, as will hereinafter be explained. Then the carrier 206 with the probes 207 is returned by X-axis displacement to a position above the matrix 202 on the table 201 and the latter is indexed to bring the sixteen probes 207 into alignment with the array of sixteen cells "2".

The plants are then transferred by the carrier 206 and probes 207 and planted into compost in the transplanting tray or blocks 204 at sixteen respective positions "2", appropriate indexing of the table 201 and the conveyor 203 and X-axis displacement of the carrier 206 being performed. Similarly for each group of sixteen cells "3" to "16" the plants therein are planted in corresponding positions "3" to "16" in the transplanting tray or blocks 204.

In this way plants from all of the 256 cells of the

0232628

tray 202 are transplanted into the transplanting tray or array of blocks 204 which is four greater in length and breadth. The appropriate indexing of the table 201 and the conveyor or bed 203 and X-axis displacement of the carrier 206 is performed automatically. This may be carried out in any convenient way. For example, the indexing may be performed by electrical or electro-pneumatic devices controlled by a micro-processor or computer. Alternatively in a simple form of apparatus, indexing of table 201 and conveyor or bed 203 and X-axis displacement of the carrier 206 may be performed manually.

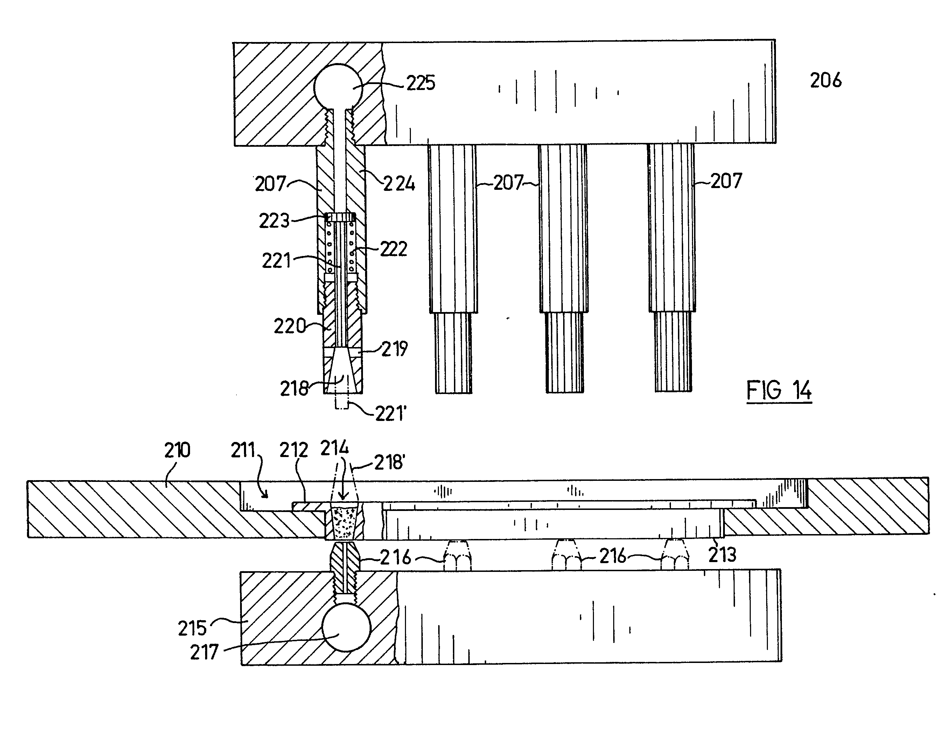

Referring now to Fig. 14, the table 201 is shown, in a preferred embodiment, in the form of a bed-plate 210 having a rectangular recess 211 therein in which is supported a cellular matrix 212 divided into, for example, a grid-like array of 16 x 16 open cells each having a bottom end defined by common gauze or mesh 213. The matrix 212 and the mesh 213 may, for example, be a plastic moulding. A single one of these cells is shown at 214.

The cells are conveniently configured as described hereinabove in connection with Figs. 5 - 7 and contain a mass of growing medium and a micro-propagated plant or seedling. Above the bed-plate 210 is disposed carrier 206 which carries an array of depending probes 207. These, as described above, are disposed in a grid-like array corresponding to the spacing required in the transplanting tray.

For example, the probes 207 are arranged in a 4 x 4 grid-like array, with each probe 207 positioned above each corresponding fourth cell 214 in the matrix 212. One of the probes 207 is shown in section.

Beneath the bed-plate 210 there is a fixed plate 215 supporting a grid-like array of air nozzles 216 at spacings corresponding to the spacings of the probes 207. When the carrier 206 is in position above the bed-plate 210, as shown in Fig. 14, each probe 207 is aligned vertically above a corresponding nozzle 216. The nozzles 216 communicate with a duct system 217 within the plate 215 through which compressed air is applied to all the nozzles 216 simultaneously, as will be hereinafter explained. The bed-plate 210 is indexed along both X and Y axes to bring every fourth cell 214 into vertical alignment with a nozzle 216 and a probe 207 in the manner described with reference to Fig. 13.

When the bed-plate 210 has been indexed to bring cells 214 into vertical alignment with the nozzles 216, as shown in Fig. 14, the bottoms of the respective cells 214 will be very close to the nozzles 216, whereby application of a jet of compressed air upwardly through the nozzles 216 will act to lift the contents of the cells 214 from the downwardly-tapered walls of the respective cells.

Each probe 207 has at its lower end or mouth a chamber 218 of frusto-conical shape tapering upwardly. Thus on lowering the carrier 206 downwardly in the Z direction, the chambers 218 of the probes will be positioned closely above the cells 214 as indicated, for one probe only, by dashed lines 218' in Fig. 14.

A tubular extension 220 of the probe 207 defines the respective chamber 218 and has vent holes 219 therein at the upper end of the chamber 218. The tubular extension 220 is closed above the vent holes 219 by a plunger or ejector pin 221 which is held in the upper position, as shown, by a helical return spring 222 acting between the upper end of the extension 220 and a piston-like head 223 on the ejector pin 221.

Thus when the probes 207 have been lowered until the lower ends of the chambers 218 abut the upper face of the cellular matrix 212 and a jet of compressed air is applied to the lower face of the corresponding cells 214, the plantlet in each of the respective cells will be pushed upwardly out of the cell 214 and transferred into the respective chamber 218, air being displaced from the chamber 218 through the vents 219. The mass of growing medium will be wedged against the frusto-conical wall of the chamber 218 such that different sizes of plants can be accommodated and held in the respective chambers 218.

The carrier 206 is then raised in the Z direction and moved in the X direction to a position above the transplanting tray or blocks 204. During this operation the plants remain held in the respective chambers 218, being wedged against the frusto-conical wall of the chamber.

The main body 224 of each of the probes 207 is also tubular and communicates with a duct system 225 within the carrier 206. Application of compressed air to the duct system 225 acts on the heads 223 of the ejector pins 221 of all the probes and pushes the ejector pins 221 downwardly, as indicated by dashed lines at 221', thereby to eject the plants from the respective chambers 218 into the compost in the transplanting tray or blocks 204. However, before this occurs, the carrier 206 is lowered to engage the lower ends of the chambers 218 into the compost or into pre-formed depressions in the compost in the tray or blocks 204 in order to effect the planting upon the ejection of the plants by the ejector pins.

The carrier 206 is moved between the position shown in Fig. 14 into the position above the transplanting tray 204 and vice versa; the probes are raised and lowered and the bed-plate 210 and the transplanting tray 204 are moved electrically or by electro-pneumatic means under the control of control means, such as a micro-processor or computer, as aforesaid, to give the required sequence of operations and indexing. Similarly the application of the jets of compressed air through the nozzles 216 to eject the plants from the cells 214 and to eject the plants from the chambers 218 on transplanting are performed electro-pneumatically under computer or microprocessor control in the correct sequence. Alternatively, the various functions described above may be performed manually by operation of suitable electrical and fluid control valves.

According to an alternative embodiment of the invention, suction may be applied to the duct system 225 to raise the ejector pins 221 at the correct times, obviating the need for springs 222.

The probes may incorporate water jets to help flush the plants out of chambers 218.

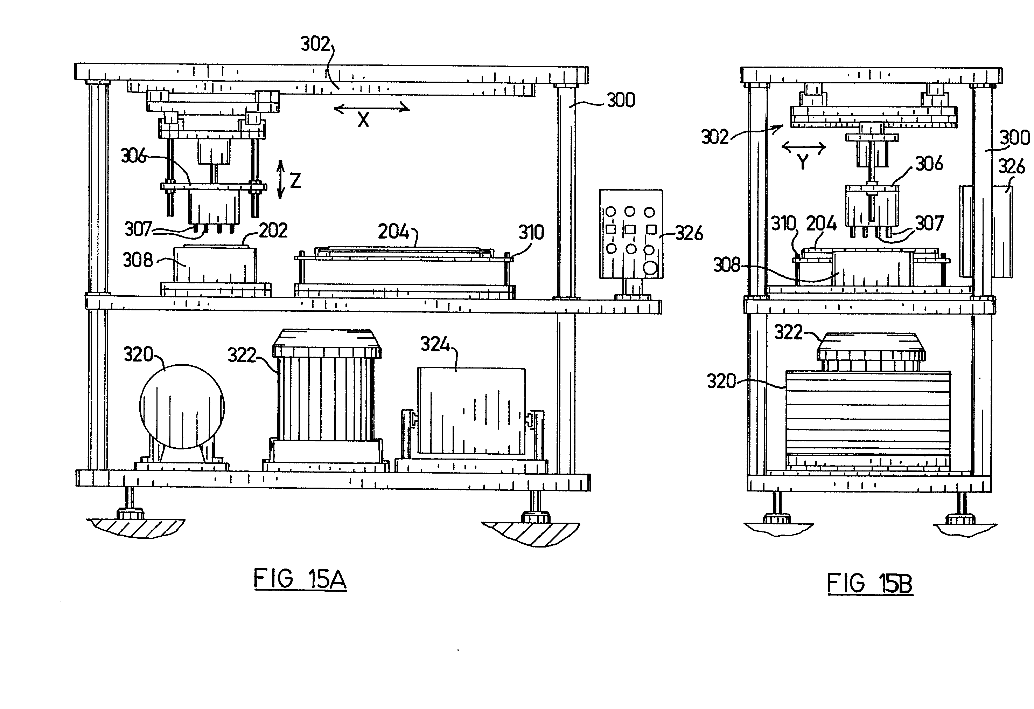

Reference is now made to Figs. 15A - 19, which illustrate an alternative embodiment of transplanting apparatus constructed and operative in accordance with a preferred embodiment of the present invention. The transplanting apparatus comprises a frame 300 onto which is mounted an X - Y gantry assembly 302. Disposed onto gantry assembly 302 is a carrier 306 having a plurality of probes 307 extending vertically downwardly therefrom and supplied by a pneumatic system, not shown in Figs. 15A and 15B.

In this embodiment of the invention, the carrier 306 can be displaced back and forth along axes designated X and Y between positions above a fixed table 308 and a fixed conveyor or bed 310 and up and down along an axis designated Z towards and away from the table 308 and the conveyor or bed 310.

Briefly stated, the essential difference between the embodiments of Figs. 11 - 14 and the embodiments of Figs. 15A - 19, lies in the fact that whereas in the embodiments of Figs. 11 - 14, transplanting is achieved by indexing of both the matrix and the transplanting tray as well as displacement of the carrier along two axes, in the embodiments of Figs. 15A - 19, indexing of the matrix and the transplanting tray is not required and all displacement is provided by the X, Y and Z axis movement of the carrier. Except as specifically described hereinabove, all of the detailed description of the structure and operation of the transplanting apparatus of Figs. 11 - 14 is applicable also to the embodiment of Figs. 15A - 19 and will not be repeated, for the sake of conciseness, identical reference numerals being used to indicate similar structure.

It is noted from a consideration of Figs. 15A and 15B that also mounted onto frame 300 is mechanical and electronic apparatus, including, for example, a compressor 320, a vacuum pump 322 and control circuitry 324, as well as a control panel 326 for enabling operator control of the transplanting function.

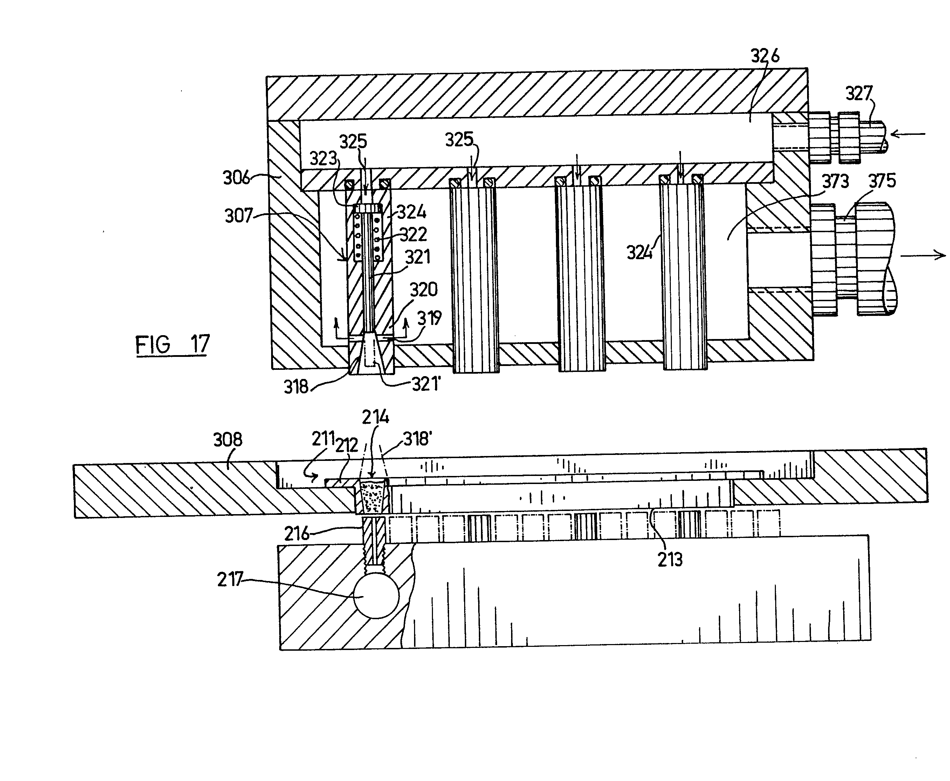

Referring now to Fig. 17, the table 308 is shown to be similar in all relevant respects to table 210 illustrated in Fig. 14 and described hereinabove, with the exception that nozzles 216 are provided under each cell 214. Therefore, as noted above, identical reference numerals are employed to designate the constituent elements of the table 308. It is appreciated that suitable manifolds are provided in communication with nozzles 216, in order to provide operation of a desired pattern of nozzles.

Above the bed-plate 308 is disposed carrier 306 which carries an array of depending probes 307. These, as described above, are disposed in a grid-like array corresponding to the spacing required in the transplanting tray.

In the illustrated embodiment of Figs. 15A - 19, the bed-plate 308 is not indexed but rather the carrier 136 is positioned in an X - Y plane so as to bring every fourth cell 214 into vertical alignment with a probe 307. The desired positioning is provided solely by suitable operation of X - Y gantry 302.

When the carrier 306 has been suitably positioned such that probes 307 lie in vertical engagement over a selected pattern of cells 214, the nozzles underlying those cells are operated to provide a jet of compressed air upwardly to lift the contents of the cells 214 from the downwardly-tapered walls of the respective cells.

Each probe 307 has at its lower end or mouth a chamber 318 of frusto-conical shape tapering upwardly. Thus on lowering the carrier 306 downwardly in the Z direction, the chambers 318 of the probes will be positioned closely above the cells 214 as indicated, for one probe only, by dashed lines 318' in Fig. 17.

A tubular extension 320 of the probe 307 defines the respective chamber 318 and has suction holes 319 formed therein at the upper end of the chamber 318. These suction holes 319 communicate with a suction volume 373 disposed within a part of carrier 306, which in turn communicates with a vacuum line 375, connected to vacuum pump 322 (Fig. 15A).

The tubular extension 320 is closed above the suction holes 319 by a plunger or ejector pin 321 which is held in the upper position, as shown, by a helical return spring 322 acting between the upper end of the extension 320 and a piston-like head 323 on the ejector pin 321.

When the probes 307 have been lowered until the lower ends of the chambers 318 abut the upper face of the cellular matrix 212 and a jet of compressed air is applied to the lower face of the corresponding cells 214, vacuum is applied via holes 319 to the chambers 318. As a result, the plant in each of the respective cells will be pushed upwardly out of the cell 214 and transferred into the respective chamber 318.

The carrier 306 is then raised in the Z direction and moved in the X - Y plane to a position above the transplanting tray or blocks 204. During this operation the plants remain held in the respective chambers 318, being wedged against the frusto-conical wall of the chamber.

The main body 324 of each of the probes 307 is also

31

tubular and communicates via ducts 325 with an air pressure manifold 326 within the carrier 306, which in turn communicates via a suitable conduit 327 with compressor 320 (Fig. 15A).

Application of compressed air to the air pressure manifold 326 acts on the heads 323 of the ejector pins 321 of all the probes and pushes the ejector pins 321 downwardly, as indicated by dashed lines at 321', thereby to eject the plants from the respective chambers 318 into the compost in the transplanting tray or blocks 204. However, before this occurs, the carrier 306 is lowered to engage the lower ends of the chambers 318 into the compost or into pre-formed depressions in the compost in the tray or blocks 204 in order to effect the planting upon the ejection of the plants by the ejector pins.

The carrier 306 is moved from the position shown in Fig. 17 to a position above the transplanting tray 204 and vice versa and the probes are raised and lowered, and pressurized air is supplied to the corresponding nozzles 216 electrically or by pneumatic or electro-pneumatic means in response to control commands provided by control means, such as a micro-processor or computer.

A preferred microprocesor for this application is a model 2800 manufactured by Control Technology Corp. of Westboro, Massachusets 01581, U.S.A. The gantry 302 typically comprises an X gantry, model number MI 81224 and a Y gantry, model number MI 80606, both manufactured by Linear Industries, Lintech Division, of 3440 San Fernando Road, Los Angeles, California 90065. The raising and lowering motion of the probes 307 is provided using linear bearings such as Model THK lM 13 manufactured by THK America of 1715 Elk Grove Village, Illinois, 60007, U.S.A. and a Model MBD 25/25 piston manufactured by Soltam of P.O.B. 371, Haifa, Israel.

Similarly the application of the jets of compressed air through the nozzles 216 to eject the plants from the cells 214 and through ducts 325 to eject the plants from the chambers 318 on transplanting and the application of vacuum via holes 319 are performed pneumatically or electro-pneumatically under computer or microprocessor control in a desired sequence. Alternatively, the various functions described above may be performed manually by operation of suitable electrical and fluid control valves.

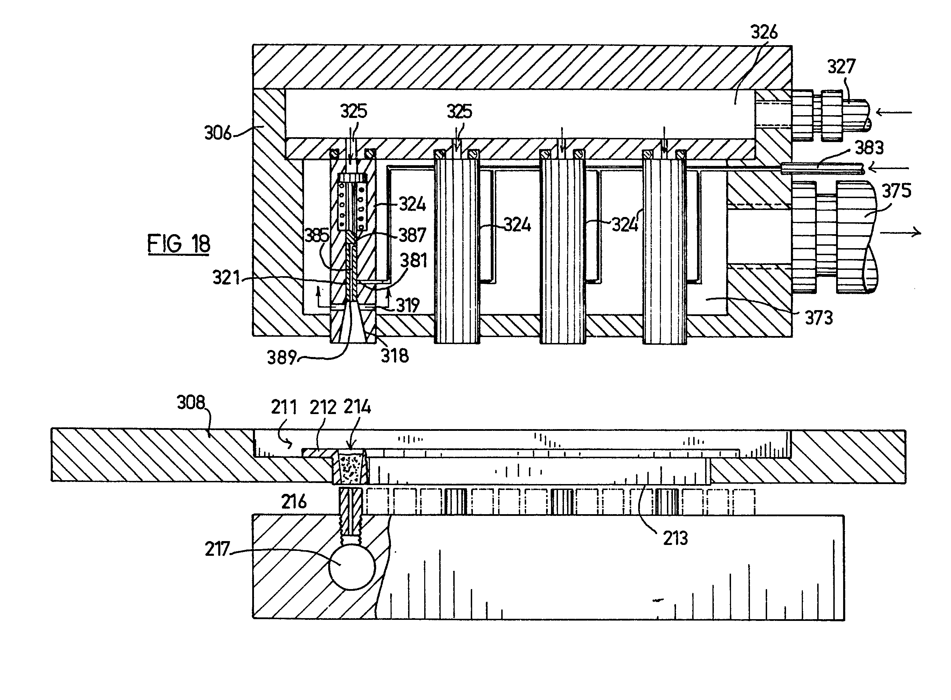

Reference is now made to Fig. 18, which illustrates an alternative embodiment of the apparatus of Fig. 17, wherein the probe is modified to provide a water jet output upon planting. Unless specifically indicated otherwise, the apparatus of Fig. 18 is identical to that of Fig. 17 and identical reference numerals are employed for identical elements.

In the apparatus of Fig. 18, extensions 320 of probes 307 are each formed with a water inlet aperture 381 which is coupled to a water supply conduit 383. Ejector pins 321 are each formed with a water pathway 385 which leads from an outlet aperture 387 at the side of the pin facing aperture 381 to an outlet aperture 389 at the butt end of the pin 321. It may be appreciated that water communication from conduit 383 via apertures 381 and 387 and pathway 385 is provided when the pin 321 is in its extended orientation, i.e. as it ejects plants during planting.

Thus, if a supply of water is provided at conduit 383, a desired quantity of water is supplied to the plant as it is ejected for flushing it out of chamber 318. It is appreciated that the timing of water supply may be governed by suitable computer control. Alternatively, the pin 321 may be arranged in sealing engagement with probe extension 320 so as to act as a valve, providing desired water output only upon extension of the pin 321 such that apertures 381 and 387 lie in registration and thus obviating the need for computer control of the water supply to conduit 383.

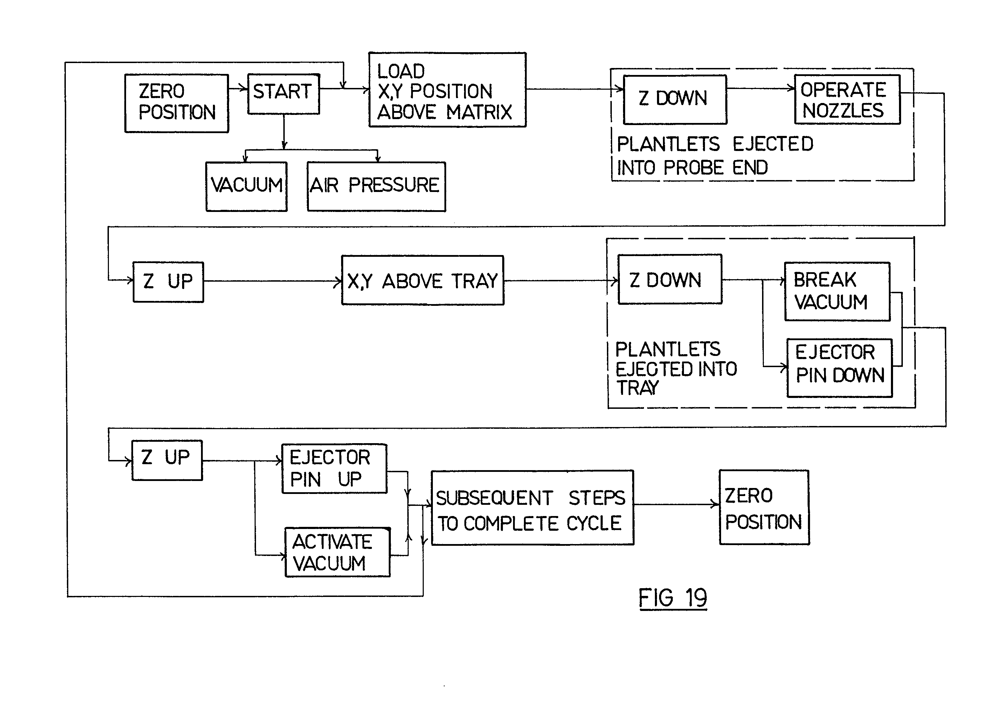

Reference is now made to Fig. 19, which is a block diagram illustration of the operation of the embodiment of the invention described in Figs. 15A to 17. Operation may be considered to begin with the carrier at its zero position, which for the sake of simplicity may be considered to be any non-operative position.

When a START command is given by the operator, using the control panel 326 (Fig. 15A), the vacuum and air pressure sources are suitably pressurized and the carrier 306 is moved to an initial load position, which, for simplicity of explanation, may be considered to be the position at which the probes 307 lie over the cells marked "1" in the arrangement illustrated in Fig. 13.

At this initial position, the probes are lowered along the Z-axis and the nozzles 216 are operated under the appropriate cells for insertion of the plantlets into the probe chambers 318. The probes are then raised along the Z-axis and the carrier is moved in the X,Y plane to a transplanting position above a transplanting tray.

Upon reaching the transplanting position, the probes are lowered along the Z-axis and vacuum is broken and the ejector pin is forced downward by pressurized air to eject the plantlet into the tray. Following ejection, the carrier is repositioned in the X,Y plane to a new position over the matrix, for example a position wherein the probes overlie the cells marked "2", the ejector pins are retracted, the vacuum is reestablished and the next array of plantlets is inserted into the probe end. The cycle continues until all of the plantlets in the matrix have been transplanted.

It will be appreciated by persons skilled in the art that the present invention is not limited by what has been particularly shown and described hereinabove. Rather the scope of the present invention is defined only by the claims which follow:

# C L A I M S

1.      A plant growth and development system comprising:

a first stage cellular growth matrix comprising a liquid permiable bottom support surface and a wall structure defining a first array of individual growing cells above said bottom support surface, said individual growing cells being separated from adjacent cells in said first array by a first distance;

a second stage growth support defining a second array of individual plantlet growth locations, said individual growth locations being separated from adjacent growth locations in said second array by a second distance greater than said first distance;

means for providing a generally even distribution of plant propagules onto said first stage cellular growth matrix in engagement with a growth medium;

means for mechanically removing individual plantlets growing in said first array of growing cells from said individual growing cells and transplanting them to corresponding individual growing locations on said second stage growth support.

2.      A plant growth and development process comprising the steps of:

providing a first stage cellular growth matrix comprising a liquid permiable bottom support surface and a wall structure defining a first array of individual growing cells

0232628

above said bottom support surface, said individual growing cells being separated from adjacent cells in said first array by a first distance;

providing a second stage growth support defining a second array of individual plantlet growth locations, said individual growth locations being separated from adjacent growth locations in said second array by a second distance greater than said first distance;

providing a generally even distribution of plant propagules onto said first stage cellular growth matrix in engagement with a growth medium; and

mechanically removing individual plantlets growing in said first array of growing cells from said individual growing cells and transplanting them to corresponding individual growing locations on said second stage growth support.

3. A process for obtaining an even distribution of plant propagules on a support and screening element disposed in a sealable vessel between upper and lower chambers thereof comprising the steps of:

introducing a liquid mixture containing plant tissue culture propagules into said upper chamber;

causing a stream of air bubbles to rise in said liquid mixture to effect agitation and mixing of said propagules in said liquid mixture above said element, thereby to form a substantially homogeneous suspension;

permitting said substantially homogeneous suspension to drain through said support and screening element to said

37

bottom chamber of said vessel, thereby causing deposit of said suspended propagules in substantially even distribution on said support and screen element.

4.      A process according to claim 2 and wherein said step of providing a generally even distribution comprises the steps of:

arranging said first stage cellular growth matrix intermediate upper and lower chambers of a vessel;

introducing a liquid mixture containing plant tissue culture propagules into said upper chamber;

causing a stream of air bubbles to rise in said liquid mixture to effect agitation and mixing of said propagules in said liquid mixture above said first stage cellular growth matrix, thereby to form a substantially homogeneous suspension;

permitting said substantially homogeneous suspension to drain through said first stage cellular growth matrix, to said bottom chamber of said vessel, thereby causing deposit of said suspended propagules in substantially even distribution on said first stage cellular growth matrix.

5.      Apparatus according to claim 1 and wherein said means for providing a generally even distribution comprises:

a sealable vessel having upper and lower chambers separated by said first stage plant growth matrix, said upper chamber comprising a controllable inlet port for introducing a liquid mixture containing plant tissue culture propagules and said lower chamber being provided with a controllable drain port and a controllable air inlet port which leads to an air bubble

0232628

distributing sparger.

6.      A cell structure for growing plant tissue culture propagules comprising a regular grid-like array of solid walled cells sized to maintain plantlets developed in each cell in substantially uniform and fixed relationship to each other.

7.      A cell structure for growing plant tissue culture propagules according to claim 6 wherein said cells are each configured as inverted truncated pyramids.

8.      A cell structure for growing plant tissue culture propagules according to claim 6 or claim 7 and also comprising a sieving and support element at the base of the cells and having a mesh size which permits the passage of a culture medium therethrough while retaining tissue culture propagules on the surface thereof.

9.      A plant growth and development system according to claim 1 and wherein said first stage cellular growth matrix comprises a regular grid-like array of solid walled cells sized to maintain plantlets developed in each cell in substantially uniform and fixed relationship to each other.

10.     A plant growth and development system according to claim 9 and wherein said cells are each configured as inverted truncated pyramids.

11.     A plant growth and development system according to claim 9 or claim 10 and wherein said liquid permiable support surface comprises a sieving and support element at the base of the cells and having a mesh size which permits the passage of a culture medium therethrough while retaining tissue culture propagules on the surface thereof.

12. A plant growth and development system according to claim 1 and wherein said cells have a width of about 5 to about 12 mm.

13. Sterilizable plant tissue culture apparatus comprising:

a sealable vessel having an inner bottom surface;

a sieving and support element disposed in said vessel and being elevated above said inner bottom surface;

culture medium filling said vessel to the level of said sieving and support element; and

tissue cultures supported on the surface of said sieving and support element in contact with said culture medium;

said sieving and support element being being provided on the surface thereof with a cell structure for growing plant tissue culture propagules comprising a regular grid-like array of solid walled cells open at the top and bottom and sized to maintain plantlets developed in each cell in substantially uniform and fixed relationship to each other.

14. Apparatus for transferring the contents of open cells arranged in a first regular grid-like array to respective positions arranged in a second regular grid-like array of which the pitch of the centers of the positions is greater than the pitch of the centers of the cells of the first array comprising:

a carrier having a plurality of probes arranged with their centers spaced apart in a regular grid-like array having a pitch substantially the same as that of the positions in the second array;

means for effecting relative bodily movement between the carrier and the first and second arrays, whereby the probes can be aligned first with cells of the first array and then with respective positions in the second array; and

means for effecting extraction of the contents of respective cells of the first array, the retention of said contents on the respective probes until the probes are aligned with respective positions in the second array, and finally the discharge of said contents into the respective positions in the second array.

15.    Apparatus for transferring plantlets from cells in a first stage cellular growth matrix, which cells are arranged in a first regular grid-like array, to respective planting locations in a second stage growing support, the planting locations being arranged in a second regular grid-like array of which the pitch of the centers of the positions is greater than the pitch of the centers of the cells of the first array by a ratio substantially equal to a multiple, comprising:

a carrier having a plurality of probes arranged with their centers spaced apart in a regular grid-like array having a pitch substantially the same as that of the positions in the second array;

means for effecting relative bodily movement between the carrier and the first and second arrays, whereby the probes can be aligned first with cells of the first array and then with respective positions in the second array; and

means for effecting extraction of the contents of respective cells of the first array, the retention of said contents on the respective probes until the probes are aligned with respective positions in the second array, and finally the discharge of said contents into the respective positions in the second array, whereby plantlets are extracted simultaneously from a plurality of cells by a plurality of probes arranged in a fixed pattern and then transplanted in a required grid-like array of positions at a pitch greater in both the length and breadth directions than the pitch of the cells on the first stage growth matrix.

16.    A system for plant growth and development according to any of the preceding claims 1, 5 and 9 - 11 and wherein said means for mechanically removing and transplanting comprises:

means for transferring plantlets from cells in a first stage cellular growth matrix, which cells are arranged in a first regular grid-like array, to respective planting locations in a second stage growing support, the planting locations being arranged in a second regular grid-like array of which the pitch of the centers of the positions is greater than the pitch of the centers of the cells of the first array by a ratio substantially equal to a multiple, comprising:

a carrier having a plurality of probes arranged with their centers spaced apart in a regular grid-like array having a pitch substantially the same as that of the positions in the second array;

means for effecting relative bodily movement between the carrier and the first and second arrays, whereby the probes can be aligned first with cells of the first array and then with respective positions in the second array; and

means for effecting extraction of the contents of respective cells of the first array, the retention of said contents on the respective probes until the probes are aligned with respective positions in the second array, and finally the discharge of said contents into the respective positions in the second array,

17.     Apparatus for transferring the contents of a cell, having an open upper end and a porous or apertured lower end, in a first position, into a second position, by using a tubular probe, the apparatus comprising;

means for effecting, in succession, relative bodily movement between the probe and means defining the first and second positions, whereby the probe can be aligned with the mouth of a cell in the first position and then with the second position; and

means to apply a pressure jet of compressed air or other fluid to the cell beneath the contents thereof when the probe is aligned with the mouth of the cell in the first position, thereby to eject the contents of the cell into the probe; and

means to apply a pressure jet of compressed air or other fluid to the probe when the latter has been aligned with the second position, thereby to eject the cell contents into said second position.

18. Apparatus according to any of claims 14 - 17 and wherein said means for effecting relatively bodily movement comprise:

means for displacing said carrier along at least first and second perpendicular axes; and

means for displacing at least one of said first and second arrays along at least a third axis perpendicular to said first and second perpendicular axes.

19. Apparatus according to any of claims 14 - 17 and wherein said means for effecting relatively bodily movement comprise:

means for displacing said carrier along first, second and third perpendicular axes.

20. Apparatus according to any of claims 14 - 19 and wherein said means for effecting discharge comprise means for providing a liquid discharge upon discharge of said contents.

FIG 1

2/14

FIG 3

FIG 2

FIG 4

FIG 5

0232628

FIG 6

FIG 7

FIG 8

FIG 9

FIG 10

FIG 11

FIG 12

0232628

FIG 13

FIG 14

0232628

FIG 15B

FIG 15A

0232628

204

302

X

202

Y

FIG 16

FIG 17

0232628

FIG 18

326 327 383 375 373 324 325 324 324 387 381 319 318 385 389 321 306

308 211 212 214 213 216 217

FIG 19

ZERO POSITION → START → LOAD X,Y POSITION ABOVE MATRIX → Z DOWN → OPERATE NOZZLES

START → VACUUM, AIR PRESSURE

PLANTLETS EJECTED INTO PROBE END

Z UP → X,Y ABOVE TRAY → Z DOWN → BREAK VACUUM, EJECTOR PIN DOWN

PLANTLETS EJECTED INTO TRAY

Z UP → EJECTOR PIN UP, ACTIVATE VACUUM → SUBSEQUENT STEPS TO COMPLETE CYCLE → ZERO POSITION

14/14

0232628

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 132 413 (MILOUDA LTD.)<br><br>* complete document * | 1,2,5,8 | A 01 G 7/00<br>C 12 M 3/00 |
| A | US-A-4 201 845 (J. FEDER et al.)<br>* complete document * | 1,2,5 | |
| A | US-A-4 531 324 (N.S. YANG et al.)<br>* abstract, figures * | 1,2,5 | |
| P,A | US-A-4 586 288 (C.F. WALTON)<br><br>* abstract, figures * | 1,2,5-11 | |
| D,A | EP-A-0 132 414 (MILOUDA LTD.)<br>* abstract * | 1 | **TECHNICAL FIELDS SEARCHED (Int. Cl.4)**<br><br>A 01 C 11/00<br>A 01 G 7/00<br>C 12 M 3/00<br>C 12 N 1/00 |

The present search report has been drawn up for all claims

| Place of search | Date of completion of the search | Examiner |
|---|---|---|
| BERLIN | 31-03-1987 | WUNDERLICH J E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82